Europäisches Patentamt

European Patent Office

Office européen des brevets

(19)

(11) **EP 0 614 909 B1**

## (12) EUROPEAN PATENT SPECIFICATION

(45) Date of publication and mention
of the grant of the patent:
**10.06.1998 Bulletin 1998/24**

(51) Int Cl.[6]: **C07J 41/00**

(21) Application number: **94301657.6**

(22) Date of filing: **09.03.1994**

(54) **Method for selective acylation of estradiol**

Verfahren zur selektiven Acylierung von Estradiol

Procédé d'acylation sélective de l'estradiol

(84) Designated Contracting States:
**BE DE FR GB NL**

(30) Priority: **12.03.1993 JP 77495/93**

(43) Date of publication of application:
**14.09.1994 Bulletin 1994/37**

(73) Proprietor: **PERMACHEM ASIA, LTD.**
**Chuo-ku Tokyo (JP)**

(72) Inventors:
• **Ohwaki, Keiji**
  **Ogasa-gun, Shizuoka-ken (JP)**

• **Niino, Hideki**
  **Yokohama-shi, Kanagawa-ken (JP)**

(74) Representative: **Haigh, Charles Roy**
**J.A. KEMP & CO.**
**14 South Square**
**Gray's Inn**
**London WC1R 5LX (GB)**

(56) References cited:
**EP-A- 0 138 153        DE-A- 2 629 657**

• **PATENT ABSTRACTS OF JAPAN vol. 014 no.**
**246 (C-0722) ,25 May 1990 & JP-A-02 067293**
**(ISHIHARA SANGYO KAISHA LTD) 7 March 1990,**

## Description

This invention relates to a method for selective acylation of the phenolic hydroxyl group at the 3-position of estradiol.

As acylation methods of the phenolic hydroxyl groups of steroids, there have hitherto been known methods using an inorganic alkali, a trialkylamine compound and a pyridine compound, respectively, as an acid acceptor in an inert solvent, and for example, it is proposed in Angew. Chem. Int. Ed. Engl., 17, 583 (1978) and EP 138,153 to use a pyridine compound selected from 4-(tertiary amino)pyridines. However, when such a known catalyst is used, hydrolysis of the reactant(s) and the product and side reactions are unavoidable, or when a strong base such as a trialkylamine compound is used as a catalyst, it often occurs that reaction does not progress at all, or if the reaction progresses, it is hard to selectively acylate only the objective phenolic hydroxyl group at the 3-position of the steroid, and there is a possibility that various complicated by-products are formed.

Further when a 4-(tertiary amino)pyridine proposed as above is used, there arises mixing of a large amount of the catalyst pyridine and its salt into the desired product after the acylation, and it is hard to isolate and purify the desired compound, and when the product is subjected to further treatments, it is seriously badly influenced due to the impurities, resulting in lowering of the yield. These are as described in GB 1,523,035. Thus, the object of this invention lies in providing a method for carrying out the acylation more efficiently.

The present inventors had carried out research on a method for solving such problems and for consistently acylating the phenolic hydroxyl group at the 3-position of estradiol, and as a result they found, unexpectedly, that when the ultra-strong base DBN or DBU or a mixture thereof is used as the acid acceptor in the above acylation, it is possible to make the acylation reaction progress conveniently with little occurrence of side reactions.

Thus the above object can be attained by a method according to this invention, namely in a method for selective acylation of the phenolic hydroxyl group at the 3-position of estradiol which comprises reacting estradiol with an N-bis(2-chloroethyl)carbamoyl halogenide in an inert solvent in the presence of an acid acceptor, wherein the improvement comprises using one of the above ultra-strong bases as the acid acceptor.

As the inert solvent according to this invention, there is used an aromatic hydrocarbon such as toluene or xylene; or a chlorinated hydrocarbon such as methylene chloride or chloroform; or a mixture of two or more of them. The ultra-strong base, used is 1,8-diazabicyclo (5,4,0)undecene-7 (trade name DBU) or 1,5-diazabicyclo(4,3,0)nonene-5 (trade name DBN) or a mixture thereof. As the N-bis(2-chloroethyl)carbamoyl halogenide, there can be used N-bis(2-chloroethyl)carbamoyl chloride or N-bis(2-chloroethyl)carbamoyl bromide.

According to the method of this invention, it is possible, by success of the new trial of using one of the above ultra-strong bases as an acid acceptor in an inert solvent, to introduce the acyl group derived from an N-bis-(2-chloroethyl)carbamoyl halogenide into the phenolic hydroxyl group at the 3-position of estradiol extremely easily and with consistent selectivity.

The method of this invention is an epoch-making method which makes it possible to obtain the pure desired product in a high yield without mixing the ultra-strong base into the desired product, and it has no bad influence on the subsequent treatment steps.

Further when the above mentioned ultra-strong base is used as an acid acceptor according to this invention, it is possible to convert the alcoholic hydroxyl group at the 17-position of estradiol to another ester group by a conventional method; preparation of the desired disubstituted ester can be carried out without difficulty; and it is possible to provide an industrial preparation method excellent for preparation of an intermediate of a useful compound such as, for example, estradiol-3-N-bis(2-chloroethyl)carbamate-17-phosphate (common name : estramustin phosphate) having an antitumor activity as a derivative thereof.

It is sufficient that the temperature for carrying out this invention is a temperature equal to or less than the boiling point of the inert solvent. The temperature is not particularly limited, but is preferably 60°C or less.

This invention is further specifically described below by examples.

Example 1

22 g of DBU and 30 g of estradiol were added to 700 ml of chloroform. Then, a solution obtained by dissolving 27 g of N-bis(2-chloroethyl)carbamoyl bromide in 200 ml of chloroform is added dropwise at 5 to 30°C under cooling and stirring to carry out the reaction. After the reaction, an oily matter obtained after washing with a suitable amount of diluted hydrochloric acid was purified by recrystallization with 800 ml of a mixed solvent of methanol and ethanol and dried to obtain 44.3 g (yield : 91.4%) of pure estradiol-3-N-bis(2-chloroethyl) carbamate. Melting point : 125.6°C.

Angle of rotation $[\alpha]_D^{20}$ +50.6° (dioxane)

Its purity by a liquid chromatography internal standard method (internal standard : isoamyl benzoate) was 99.6%.

Example 2

3.4 g of DBN and 6.0 g of estradiol were added to 133 ml of methylene chloride. Then, a solution obtained by dissolving 6.5 g of N-bis(2-chloroethyl)carbamoyl bromide in 45 ml of methylene chloride was added dropwise at 0 to 20°C under cooling and stirring to carry out the reaction.

The subsequent operations were carried out in the same manner as in Example 1 to obtain 8.7 g (yield : 89.7%) of the same pure product.

Example 3

The same operations as in Example 1 were carried out except that chloroform was replaced by toluene to obtain 42.0 g (yield : 86.6%) of the same pure product.

Example 4

The same operations as in Example 1 were carried out except that methylene chloride of Example 2 was replaced by xylene and 5.5 g of N-bis(2-chloroethyl)carbamoyl chloride was used to obtain 8.2 g (yield : 84.5%) of the same pure product.

Example 5

2.2 g of DBU and 3.0 g of estradiol were added to a mixed solvent of 50 ml of chloroform and 100 ml of methylene chloride.

Then, a solution obtained by dissolving 13.5 g of N-bis(2-chloroethyl)carbamoyl chloride in 100 ml of chloroform was added dropwise under cooling and stirring to carry out the reaction.

The subsequent operations were carried out in the same manner as in Example 1 to obtain 4.3 g (yield : 87.7%) of the same pure product.

Example 6

6 g of DBU, 5.1 g of DBN and 15 g of estradiol were added to 350 ml of chloroform. Then, a solution obtained by dissolving 13.5 g of N-bis(2-chloroethyl)carbamoyl chloride in 100 ml of chloroform was added dropwise under cooling and stirring to carry out the reaction.

The subsequent operations were carried out in the same manner as in Example 1 to obtain 20.8 g (yield : 86%) of the same pure product. Its purity by liquid chromatography was 99.6%.

**Claims**

1. A method for selective acylation of the phenolic hydroxyl group at the 3-position of estradiol which comprises reacting estradiol with an N-bis(2-chloroethyl)carbamoyl halogenide in an inert solvent chosen from aromatic hydrocarbons and chlorinated hydrocarbons or a mixture of two or more of them in the presence of an acid acceptor, characterized in that an ultra-strong base chosen from 1,8-diazabicyclo(5,4,0)undecene-7, or 1,5-diazabicyclo(4,3,0)nonene-5 and a mixture thereof is used as the acid acceptor.

2. The method according to claim 1 wherein toluene, xylene, methylene chloride or chloroform are used alone or in admixture as the inert solvent.

3. The method according to any one of claims 1 or 2 wherein the halogen of the N-bis(2-chloroethyl)carbamoyl halogenide is chlorine or bromine.

**Patentansprüche**

1. Verfahren zur selektiven Acylierung der Phenolhydroxylgruppe an der 3-Position von Estradiol, umfassend das Umsetzen von Estradiol mit einem N-Bis(2-chlorethyl)carbamoylhalogenid in inerten Lösungsmittel, gewählt aus aromatischen Kohlenwasserstoffen und chlorierten Kohlenwasserstoffen oder einem Gemisch von zwei oder mehreren von diesen, in Gegenwart eines Säureakzeptors, dadurch gekennzeichnet, daß eine ultrastarke Base, gewählt aus 1,8-Diazabicyclo(5,4,0)undecen-7 oder 1,5-Diazabicyclo(4,3,0)nonen-5 und einem Gemisch davon als der Säureakzeptor verwendet wird.

2. Verfahren nach Anspruch 1, wobei Toluol, Xylol, Methylenchlorid oder Chloroform einzeln oder in Gemischen als das inerte Lösungsmittel verwendet werden.

3. Verfahren nach einem der Ansprüche 1 oder 2, wobei das Halogen des N-Bis(2-chlorethyl)carbamoylhalogenids Chlor oder Brom ist.

**Revendications**

1. Procédé d'acylation sélective du groupe hydroxyle phénolique placé en position 3 de l'oestradiol, qui comporte le fait de faire réagir de l'oestradiol avec un halogénure de N-bis(2-chloroéthyl)carbamyle, dans un solvant inerte choisi parmi les hydrocarbures aromatiques et les hydrocarbures chlorés ou dans un mélange de deux de ces solvants ou plus, en présence d'un accepteur d'acide, lequel procédé est caractérisé en ce que l'on emploie, comme accepteur d'acide, une base ultra-forte choisie parmi le 1,8-diazabicyclo[5.4.0]undécène-7, le 1,5-diazabicyclo[4.3.0]nonène-5 et un mélange de ceux-ci.

2. Procédé conforme à la revendication 1, dans lequel on emploie, comme solvant inerte, du toluène, du xylène, du chlorure de méthylène ou du chloroforme, seuls ou mélangés.

3. Procédé conforme à l'une des revendications 1 et 2, dans lequel l'halogène contenu dans l'halogénure de N-bis(2-chloroéthyl)carbamyle est du chlore

ou du brome.